# EUROPEAN PATENT SPECIFICATION

(11) **EP 0 406 931 B1**
(45) Date of publication and mention of the grant of the patent: **15.03.1995**
(21) Application number: 90201609.6
(22) Date of filing: 20.06.1990
(51) Int. Cl.: C07K 5/02, C07K 7/02

(54) **New analogues of all-bond retroinverted thymopentin, the method for the synthesis of the same and their employment for the preparation of pharmaceutical compositions**
Thymopentin-Analoge mit allen umgekehrten Verbindungen, Verfahren zur deren Herstellung und deren Verwendung zur Herstellung von pharmazeutischen Zusammensetzungen
Analogues de la thymopentine avec toutes les liaisons rétro-inverties, leur méthode de préparation et leur utilisation pour la préparation de compositions pharmaceutiques

(30) Priority: 30.06.1989 IT 2103789
(43) Date of publication of application: 09.01.1991
(73) Proprietor: SCLAVO S.p.A., I-53100 Siena (IT)
(72) Inventor: Mariotti, Sabina, I-02032 Fara Sabina, Rieti (IT); Sisto, Alessandro, I-00136 Rome (IT); Nencioni, Luciano, I-53036 Poggibonsi, Siena (IT); Villa, Luigi, I-50124 Florence (IT); Pessi, Antonello, I-00199 Rome (IT)
(74) Representative: Gervasi, Gemma, Dr.

(56) References cited:
- PHARMACOLOGICAL RESEARCH COMMUNICATIONS, vol. 20, suppl. 2, 1988; A. SISTO etal.: "Biologically active retro inverted analogues of thymopentin", &XXIVTH NATIONAL CONGRESS OF THE ITALIAN SOCIETY OF FARMACOLOGY, Brescia, 25th - 28 September 1988

## Description

This invention relates to new analogues of thymopentin (TP5), all amide bonds of which have been retro-inverted, as well as to the process for the preparation of such new compounds and their employment in pharmaceutical compositions. In particular, a first object of the present invention consists in the thymopentin analogues having the following general formula (I): 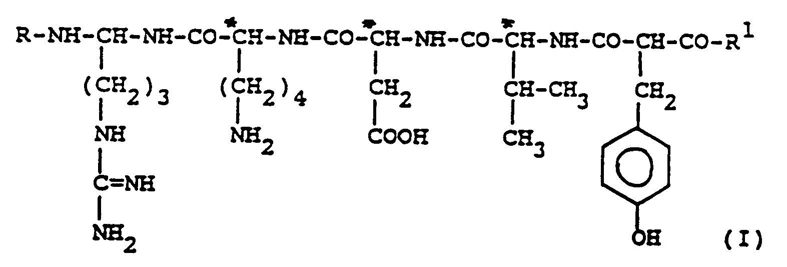
wherein R is hydrogen or an acyl radical, which is metabolically labile or whose bond with the amino group is rapidly cleaved in the first steps of the metabolic path of the product,
R¹ is a -OR² group wherein R² is a hydrogen atom or an alkyl radical having a straight or a branched chain containing from 1 to 6 carbon atoms, an alkenyl or alkynyl radical with a straight or a branched chain containing from 3 to 6 carbon atoms, or an aryl-alkyl or alkyl-aryl radical containing from 7 to 12 carbon atoms, the carbon atom configuration pointed out by an asterisk being of the D-configuration,
and the corresponding acid or basic addition salts thereof which are pharmaceutically acceptable.

The new compounds of the present invention defined above by means of the chemical structural formula that distinguishes the same can be pointed out more concisely employing the symbols that are internationally recognized in the peptide field, as:
R-H-gArg-D-Lys-D-Asp-D-Val-mTyr-R¹ (I)
wherein R and R¹ have the same meanings as those mentioned above.

In such formulas, gArg points out a geminal diamino residue that can be derived from the amino acid arginine by substitution of an amino group for the carboxyl group, while mTyr points out a malonyl residue which is substituted in the 2-position with the side chain of the amino acid tyrosine. As regards the objects of the present invention, the expression "acyl radical" points out the acyl radicals deriving from alkanoic acids having a straight or a branched chain and containing from 1 to 6 carbon atoms, as for instance the formyl, the acetyl, the propionyl, the succinoyl radical and so on, and the aromatic acyl radicals deriving from benzoic acid and from substituted benzoic acid, as for instance the benzoyl, the 4-nitro-benzoyl, the 2,3,4-trimethoxybenzoyl radical etc. As the expression "pharmaceutically acceptable salts" is employed herein, it points out those acid or basic addition salts in which the anion or the cation respectively are relatively non-toxic and innocuous when said compounds are administered, just in the form of their addition salts, at dose rates which are therapeutically efficient, so that any possible side effects of said anions or cations do not affect badly the advantageous effects of the active principle.

Among the acids that are capable of forming acid addition salts which are pharmaceutically acceptable with the compounds of formula (I), the inorganic acids can be mentioned as a particular instance, such as hydrochloric acid, hydrobromic acid, phosphoric acid, sulfuric acid, etc., the carboxyl organic acids as for instance formic acid, acetic acid, propionic acid, lactic acid, malonic acid, succinic acid and so on, and the sulfonic organic acids, as for instance methansulfonic acid or naphthalensulfonic acid.

Bases which are capable of forming pharmaceutically acceptable salts with the compounds of formula I comprise for instance the mineral bases, like sodium or potassium hydroxide, ammonium hydroxide etc., and the organic bases like for instance triethylamine, triethanolamine, etc.

Such addition salts can be obtained directly through the process of synthesis of the new retro-inverted peptides, or they can be prepared according to standard procedures starting from the compounds of formula (I) in the form of the free bases or acids, by treatment with one or more equivalents of the acid or of the base selected. If desired, it is also possible to convert a given acid addition salt into another one, by treating the first salt with a suitable ion exchange resin as disclosed for instance by R.A. Boissonas at al., in Helv.Chim.Acta, 43, 1349 (1960).

A preferred group of compounds of the present invention comprises those compounds of the formula (I) wherein R is a hydrogen atom or an acyl radical which is metabolically labile or an acyl radical whose bond with the amino group is rapidly cleaved in the first steps of the metabolic path of the product, and which do not exert toxic or anyway therapeutically contraindicated effects at the concentration at which the compound of the formula (I) performs its pharmaceutical effect as desired, R¹ is as defined above, and R² is hydrogen.

A group which is most preferred among the compounds mentioned above comprises those compounds of the formula (I) wherein R and R¹ are as defined above and R² is hydrogen.

The compounds of the present invention have shown to possess a remarkable activity on the immune system.

In the last fifteen years, G. Goldstein and his group have studied in detail the biological action and the possible pharmacological exploitation of a polypeptide hormone secreted by thymus epithelial cells, i.e., thymopoietin (G. Goldstein, Nature, 247, 11 (1974); D.H. Schlesinger et al., Cell, 5, 361 (1975); T. Audhya et al., Biochemistry, 20, 6195 (1981), whose primary sequence is made up of 49 amino acids.

Thymopoietin performs a number of various regulating actions in the organism, by affecting neuromuscular transmission (G. Goldstein, Lancet, 2, 119 (1968), the differentiation of lymphocytes T and B (M.P. Scheid et al., J.Exp.Med., 147, 1727 (1978)) and the immune response (C.Y. Lau et al., J.Immunol., 125, 1634 (1980)).

Studies about structure-activity relationships have shown that the whole sequence of 49 amino acids of thymopoietin is not necessary for biological activity, as the pentapeptide
H-Arg-Lys-Asp-Val-Tyr-OH
(thymopentin-TP5) corresponding to the 32-36 amino acid sequence, has the full biological activity of the natural hormone both in vitro and in vivo (G. Goldstein et al., Science, 204, 1309 (1979).

Thymopentin has already been introduced in the clinical practice both in the treatment of autoimmune origin diseases, as for instance in the treatment of rheumatoid arthritis and as a stimulating agent of the organism defence system for instance in the treatment of primary immune deficiencies caused by the absence or by the incomplete development of thymus with the consequent alteration in the maturation of T lymphocytes, and in the treatment of acute or recurrent viral diseases, or also as an adjuvant in vaccinations.

Anyway, the setting forth of a therapeutic standard treatment is made critical by difficulties deriving from the determination of the efficient dose, as the pharmacological effect can change strongly with the procedure and the duration of the administration (T. Audhya et al., Surv.Immunol.Res., 4^{th} Suppl.,1, 17 (1985); and T.Audhya et al., Int.J.Peptide Protein Res., 22, 568 (1983). The limiting example is that reported by Bolla et al., in Int.J.Clin.Pharm.Res., IV(6), 431, (1984), according to which the effect that stimulates the production of antibodies on the part of TP5 administered subcutaneously is completely suppressed if the same dose is administered by the intravenous route. A possible cause of this is the short half-life of TP5 in the plasma. The TP5 indeed becomes rapidly degraded in the plasma (t_{1/2} = 1.5 min) by the action of various proteases. (T.P. Tischio et al., Int.J.Peptide Protein Res., 14, 479 (1979)).

An intense research activity has been undertaken in the recent years in order to obtain TP5 analogues having increased resistance to proteases. For instance, the European Patent Application EP-A-135722 and the US Patent No. 4,505,853 can be referred to, which patents relate to thymopentin analogues obtained by opportunely varying the single amino acids inside the peptide sequence.

Again in order to obtain such object, a series of thymopentin analogues have been recently synthesized which contain one or more retro-inversion at the bonds which are more labile to enzymic hydrolysis of the peptide chain (cf. EP-A-282.891 and the European patent application Publ. No. 0375058 and Pat. Appln. No. 90200954.7).

The compounds so obtained that keep the immunomodulation activity of TP5 unaltered or even improved, have shown to be much more stable with respect to the proteolytic enzymes of the natural peptide. In particular, for instance, while the half-life of TP5 in heparinized human plasma turned out to be of 1.5 minutes, that of the (1,2) r.i. TP5 analogue has turned out to be of 22 minutes.

Even longer half-lives have been obtained by modifying the TP5 molecule by retroinversion of two amide bonds.

The two (1,2-3,4) r.i. TP5 and (1,2-4,5) r.i. TP5 analogues, though keeping the same immonostimulating activity as that of TP5, turn out to be particularly stable to enzymic demolition, with half-lives longer than 6 hours.

On the other side, the retroinversion of two non-adjacent peptide bonds gives rise during the synthesis of the two analogue compounds to the conversion of two amide functions into amine functions, and to the preparation of two 2-substituted malonic derivatives.

Moreover, the final product is obtained as a mixture of four isomers, by the introduction of two malonic residues into the pseudopeptide sequence, as racemic products.

The resolution of the isomers, if desired, would ask for complicated preparative chromatographic steps.

It has now been found surprisingly that by inverting all bonds in the TP5 molecule, i.e., by substituting the corresponding geminal diamino residue for the N-terminal amino acid arginine, as well as the corresponding 2-substituted malonyl residue for the C-terminal amino acid tyrosine, and inserting all other amino acid residues into the inverted-configuration sequence, a product is equally obtained showing an immunostimulating activity stronger than that of TP5, and a stability to proteases which is comparable to that of the two pseudopeptides mentioned above.

For the preparation, a less number of critical chemical steps is also necessary, and, above all, the product is obtained as a mixture of two only diastereoisomers which are more easily separable, if desired, by HPLC (High Pressure Liquid Chromatography).

The compounds of the present invention are easily prepared from the corresponding compounds of formula Ia) 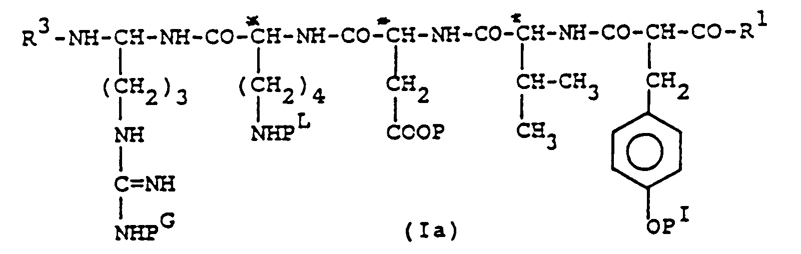
wherein:
R³ is R or a group which protects the alpha-amino function,
P^{G} is a suitable protective group of the guanidino function,
P^{L} is a protective group of the amino function in the side chain,
P is a protective group of the carboxyl function,
P^{I} is a protective group of the hydroxyl function of tyrosine,
R¹ is as defined above,
while the configuration of the asymmetric carbon atoms marked with an asterisk is the D-configuration.

The removal of such protective groups is carried out according to procedures which are well known in the art. In general, when conventional protective groups are employed such as the tert-butyl or tert-amyl for the carboxyl and the hydroxyl group of tyrosine, the tert-butoxycarbonyl or benzyloxycarbonyl group for the amino group of lysine, and the benzensulfonyl groups for the guanidino group of arginine, these are conveniently removed by acidolysis in an acid medium as for instance hydrochloric acid diluted in acetic acid, or with trifluoroacetic acid or with mixtures of trifluoroacetic acid and trifluoromethansulfonic acid, in the presence of small percentages of ethandithiole, anisole, thioanisole or resorcinol which are employed as "scavengers" for trapping the carbocations which are formed.

At the end of the reaction, the desired product of the formula (I), as such or in the form of an addition salt, is recovered and then purified employing conventional procedures.

The homogeneity of the compounds so obtained is tested by tlc and HPLC while their purity is determined through amino acid analysis and NMR.

The compounds of the formula (Ia) wherein R³ is R, can be obtained by treatment with I,I-bistrifluoroacetoxy-iodobenzene (TIB) of the corresponding terminal amides of formula (IV): 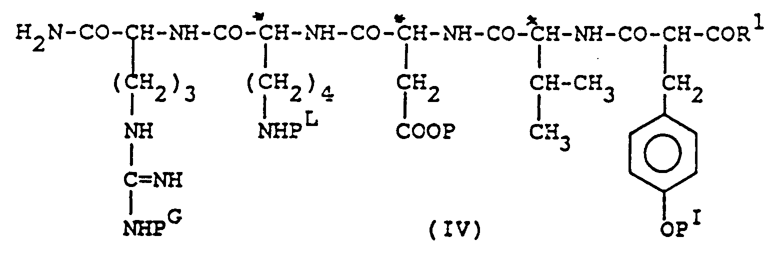
said treatment being possibly followed by acylation of the terminal amino function so formed. The intermediates of formula (IV) in their turn can be prepared by successive condensations of an amide of the formula (V): 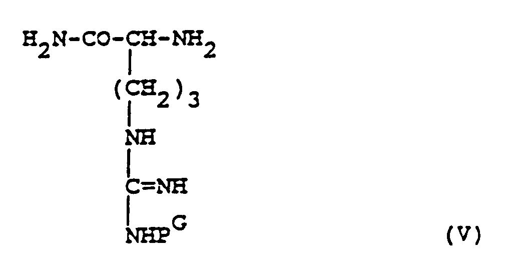
wherein P^{G} is as defined above, with a residue of D-lysine, of D-aspartic acid and of D-valine, which are protected in a suitable way so giving rise to intermediate compounds of the general formula (VI) 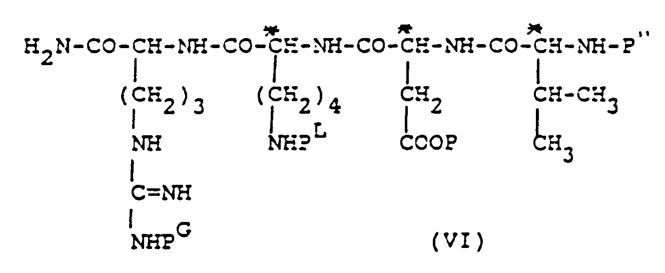
wherein P, P^{G}, P^{L} have the meanings mentioned above, the configuration of the carbon atoms marked with an asterisk being D-, and P˝ is hydrogen or a protective group of the alpha-amino function, which are easily removable under conditions which do not cause the other protective groups to be removed,
which is then caused to react, once the transitory protective group P˝ is removed, with a Meldrum's acid suitably substituted in the 5-position with the side chain of the amino acid tyrosine protected at the phenolic group with a protective group P^{I} (VII): 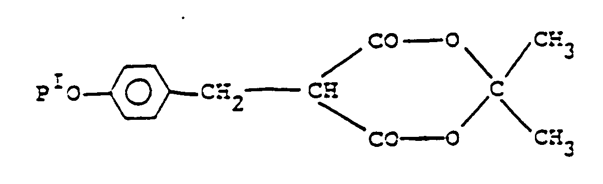
in the presence of a silanizing agent.

As regards the first condensations, these can be conveniently performed according to any one of the methods already known in the literature for the peptide synthesis. Optimum results have been obtained any way in terms of yields and purity of the products by employing a carbodiimide such as dicyclohexylcarbodiimide or diisopropylcarbodiimide and 1-hydroxybenzotriazole. For such condensation reactions, which can be conveniently carried out at room temperature, the conventional aprotic polar organic solvents are employed which are capable of solubilizing the reactants and do not interfere negatively with the reaction behaviour. Solvents of choice are dimethylformamide, acetonitrile, dimethylsulfoxide, possible mixed with less polar solvents such as for instance halogenated aliphatic hydrocarbons, as for instance methylene chloride, dichloroethane, and so on.

Protective groups that are conveniently usable in that synthesis are those which are conventionally employed and well known in the technical literature, and commonly used in classical peptide syntheses. In particular, P^{L} is preferably a tert-butoxycarbonyl group or a benzyloxycarbonyl group, possibly nitro- or halogeno-substituted; P^{G} is preferably a benzensulfonyl group substituted in various ways, as for instance an alkylbenzensulfonyl group, e.g. like toluensulfonyl, or an alkyl-alkoxybenzensulfonyl group, e.g. the 4-methoxy-2, 3,6-trimethylbenzensulfonyl group; and finally P can be any protective group of the terminal carboxyl functions like for instance an alkyl group, e.g. the tert-butyl or tert-amyl group, or an arylalkyl group, e.g. the benzyl or substituted benzyl group.

When the sequence of such condensation reactions, whose behaviour can be quite easily followed by tlc, is complete, the product so obtained corresponding to the formula (VI) is recovered employing conventional procedures.

More particularly, when according to a preferred embodiment a carbodiimide is employed as a "coupling agent", such standard procedures contemplate the separation of the urea so formed by filtration, the evaporation of the solent, the washing of the residue or of a solution of the same in a suitable organic solvent, with weakly basic or weakly acid solutions, these steps being followed by the final purification of the product by crystallization or chromatography.

Once the transitory protective group P˝ is removed, the intermediate compound is contacted with the derivative of Meldrum's acid (VII) in the presence of a silanizing agent according to what has been disclosed in the European Patent Appln. Publ. No. 0375040, the contents of which have been incorporated herein just for reference.

Finally the reaction of the product so obtained corresponding to the formula (IV) with TIB is then carried out according to the procedures disclosed in the European Patent Appln. Publ. No. 084691 which procedures contemplate the reaction of the amide substrate with a slight excess of TIB in water mixtures of inert organic solvents such as for instance dimethylformamide, acetonitrile etc. The reaction is carried out by bubbling through the reaction mixture an inert gas, typically nitrogen, checking the behaviour of the reaction by tlc. When the complete conversion of the amide into the amine has been observed, the organic solvent is removed and the product is easily recovered by lyophilization.

Accordingly, further objects of the present invention consist in the process for the synthesis of the new compounds, as well as in the new intermediate compounds of the formulas (Ia), (IV) and (VI) which are obtained in such procedure.

The compounds of the present invention can exist in a number of isomeric forms.

Indeed, there are at least five asymmetric carbon atoms in the structural formulas (I), but the absolute configuration of the amino acids interposed between the gem-diamino derivative and the malonyl residue (those which are marked with an asterisk) is the D-configuration (and such previously fixed configuration is obtained by merely employing in the synthesis the suitable D-amino acid derivatives), the absolute configuration of the gem-diamino carbon atoms is also fixed as the gem-diamino fragments are obtained starting from the corresponding D-amino acid amides, while the carbon atoms of the malonyl residues can be of the R or S configuration. Accordingly, the compounds of the present invention are obtained as a mixture of the two diastereoisomers which can any way be separated, if desired, into the single isomers according to the well known resolving methods.

The compounds of the formula (I) can any way be employed both as their optically active form and as their form of a mixture of the isomers.

The compounds of the present invention showed to be more stable than thymopentin to the action of plasma peptidases.

In particular, the lability to enzymic hydrolysis of [gArg¹,D-Lys²,D-Asp³,D-Val⁴,(R,S)mTyr⁵]TP5 (c.r.i.)TP5, has been tested in a parallel way to the TP5, employing heparinized human plasma and incubating in the same separately the peptides at the concentration of about 30 nmoles/ml of the plasma. The incubation has been carried out at 37°C, and the samples of the plasma mixtures of 100 »l each drawn have been blocked at the various times by adding trifluoroacetic acid in the amount of 10 % and successive centrifugation at 10,000 x g for five minutes. A portion of the supernatant is chromatographed under conditions which are suitable to put into evidence the change in the concentration of the peptide tested at the various times. From the enzymic kinetics so obtained, the half-life was calculated, i.e. the incubation time at 37°C required for degrading 50 % of the peptide tested, which is reported in the following Table I.

**Table I**

| Stability to enzymic hydrolysis in human plasma | |
|---|---|
| | (t_{1/2} in minutes) |
| TP5 | 1.5 |
| (c.r.i.) TP5 | 360 |

The higher stability of the new retro-inverse analogue compounds with respect to TP5 has also been put into evidence by employing isolated enzymes (leucinaminopeptidase and carboxypeptidase).

The immunostimulating activity of such new analogues has been tested in vivo in comparison to that of TP5.

In particular, the so-called haemolysis plate test has been employed (PFC) carried out according to the procedure disclosed by Jerne and Nordin.

The test is carried out by administering through intravenous route to inbred male mice C3H/HeNCrlBr (Calco-Italia), 10-12 weeks old, and of body weight of about 25 g, a pyrogen-free saline solution (0.2 ml) containing 1-2 x 10⁸ ram red blood cells (SRBC). After two hours, groups of three mice each are treated per os (intra-gastric intubation) with 0.2 ml of saline solution (the control subjects) or with 0.2 ml of saline solution containing the peptide under test. After 4 days, said mice are sacrificed and the spleens are drawn and mechanically disrupted for separating the lymphocytes. The lymphocytes so isolated are washed with minimum soil containing Earle's salts (MEM) (3 x 15 ml) and then suspended again throughout the same soil (1 ml) at a final concentration of 150,000 cells. Each cell suspension (0.1 ml) is then diluted in the ratio 1/100 with MEM soil, and 100 »l of each dilution are added in double sampling into the wells of microplates containing 25 »l of MEM soil, 25 »l of a 10 % solution of SRBC antigens and 25 »l of guinea pigs complement at a final dilution of 1/64. The whole suspension is immediately transferred by capillarity from well into overlapped glass slides. Such slides are then sealed with paraffin along their edges, then incubated within a thermostat at 37°C for one hour.

At the end of that period, the direct haemolysis plates are counted by means of a light-contrast viewer, said plates pointing out the number of lymphocytes that secrete antibodies (PFC). The antibodies so put into evidence are of the IgM class and are proper of the primary antibody response. The results expressed as the percentage with respect to the control subjects, are reported in the following Table II:

**Table II**

| Compound | Plate Formation % of the control |
|---|---|
| c.r.i.-TP5 | |
| 1 ng | 219 |
| 10 ng | 170 |
| 100 ng | 144 |

In Table II as well as in other parts of the present disclosure, "c.r.i." means "completely retro-inverted".

Because of such biological characteristics, the peptides of the present invention are capable of interfering with the body immune response, stimulating the same substantially when it is deficient. Accordingly, the compounds of the present invention are therapeutically useful in the treatment of a set of pathological states which are connected to an immune deficiency. Among such states, there is for example the DiGeorge syndrome which is characterized by a congenital absence of thymus, or the infections of viral nature, of the fungus or mycoplasmatic type, chronic or of long duration.

Accordingly, a further object of the present invention consists in pharmaceutical compositions containing a therapeutically efficient amount of one or more compounds corresponding to the formula (I).

For therapeutic employment as immunostimulating or immunomodulating agents, the compounds of the present invention can be conveniently administered through the parenteral, the oral or the sub-lingual route. The formulations containing the new compounds can be prepared according to the standard procedures by combining the active principle with an inert vehicle and possibly with some conventional additives selected in a suitable way.

For the oral or sub-lingual employment, the compounds of the present invention can be administered in the form of tablets, capsules, drops, elixir, etc., which are prepared employing the conventional carriers/excipients such as starch, sugars, water, alcohol and so on, and possibly containing flavouring agents, stabilizing agents, preserving agents and lubricants, etc. For parenteral employment, the carrier of choice is sterile injection water. Additives can be introduced according to the well known techniques. The daily dose rate which is therapeutically efficient will change according to the subject to be treated (weight, age and condition), and according to the administration route. However,in general the compounds of the present invention are active when they are administered at a daily dose rate between 2 and 200 ng/kg. The pharmaceutical formulations of the present invention will thus contain the compounds of the formula I in a suitable amount to ensure a correct daily dose rate inside the range pointed out above.

The following examples disclose in detail some representative compounds of the present invention and the method for the synthesis thereof.

### Example 1

### [gArg¹, D-Lys², D-Asp³, D-Val⁴, (R, S)mTyr⁵]TP5

### 1) Fmoc-D-Arg)(Mtr)-NH₂

A solution of the ammonium salt of N-hydroxybenzotriazole (HOBt) (2.49 g, 15.26 mmoles) in dimethylformamide (DMF) (15 ml) and a solution of dicyclohexylcarbodiimide (DCC) (3.21 g, 15.6 mmole) in DMF (15 ml) are added to a solution of Fmoc-D-Arg(Mtr)-OH (7.9 g, 13.0 mmoles) in DMF (50 ml) cooled to 0°C and under stirring.

After about one hour the temperature is allowed to rise up to the value of the ambient temperature and the stirring is continued for an additional hour.

Then the DCU so formed is filtered off and the solvent is evaporated so as to obtain an oily residue which is taken with AcOEt (70 ml) and washed first with a water solution of 5 % NaHCO₃ ( 3 x 50 ml) and then with a saturated water solution of NaCl (3 x 50 ml).

The organic solution is then dried over MgSO₄ and then taken to dryness. The solid residue so obtained is triturated with Et₂O (100 »l) so giving a colourless powder (7.6 g, yield 96 %).
Melting point 168-172°C.
tlc (chloroform/methanol/acetic acid 85/10/5) (CMA) Rf = 0.44
HPLC - column: Hibar, Lichrosorb^{R} RP-18 (10 »).
Eluting agents: gradient from 0 to 40 % of the solution B in A during the first 20 minutes, from 40 to 80 % of B in A in the next 10 minutes and then at a constant value of 80 % of B for further 5 minutes (B = 0.1 % TFA in CH₃CN; A = 0.1 % TFA in 10 % CH₃CN in H₂O).
Flowrate: 1.5 ml/min
Detection: UV at 230 nm
Single peak with T_{R} = 26.19′

### 2) H-D-Arg(Mtr)-NH₂.HCl

A suspension of the compound of the preceding step (7.9 g, 13.0 mmoles) in a DMF/diethylamine mixture 80/20 (100 ml) is kept stirred for one hour. Then the solvent is evaporated off under reduced pressure and the residue is taken with AcOEt (100 ml). The organic solution is then extracted with a 0.1 N water solution of HCl (3 x 50 ml) and the water extracts combined are washed with 50 ml of AcOEt and then lyophilized a number of times so obtaining a flaky produce (3.25 g, yield 65 %).

The product has no exact melting point.

¹H-NMR analysis confirms the structure assigned to the product.

The HPLC analysis in the conditions of step 1) puts into evidence a single peak with T_{R} = 12.69′.
tlc (butanol/H₂O/acetic acid 4/1/1 - (BWA)), Rf = 0.36.

### 3) Z-D-Lys(Boc)-D-Arg(Mtr)-NH₂

A solution of 1-hydroxybenzotriazole (HOBt) (1.17 g, 8.16 mmoles) in CH₂Cl₂ (30 ml) and a solution of N, N′-dicyclohexylcarbodiimide (DCC) (1.54 g, 7.48 mmoles) in 5 ml of CH₂Cl₂ are added to a solution of Z-D-Lys(Boc)OH (2.85 g, 7.48 mmoles) in CH₂CL₂ (30 ml) cooled to 0°C and kept stirred under a nitrogen blanket. After 30 minutes, the ice bath is removed and the stirring is continued for additional 30 minutes. Then a solution of the compound obtained in step 2) (2.62 g, 7.48 mmoles) in 30 ml of a CH₂Cl₂/DMF mixture (1/2, volume/volume) is added.

After one hour, when the end of the reaction has been checked by chromatographic test, the N,N′-dicyclohexylurea (DCU) so formed is filtered off and the filtrate is taken to dryness. The residue so obtained is then taken with tetrahydrofuran (THF) (40 ml) and left standing overnight at -20°C. The solid precipitate is then removed by filtration and the THF is evaporated off under reduced pressure. The residue is then taken with AcOEt (70 ml) and treated with a 5 % NaHCO₃ water solution (50 ml) for 20 minutes at room temperature, then the organic phase is separated, washed with the same solution (2 x 50 ml), and then with a saturated water solution of NaCl (3 x 50 ml) with a 0.1 N solution of HCl (3 x 50 ml) and finally again with a saturated water solution of NaCl (3 x 50 ml).

The organic phase is then dried over MgSO₄ and taken to dryness leaving behind an off white solid of glassy consistence, in an amount of 4.90 g (yield 93.5 %).

The ¹H-NMR and mass spectrometric analyses confirm the structure assigned to the product.

The HPLC analysis in the conditions of the step 1) points out a single peak with T_{R} = 25.5′.
tlc (CMA) Rf = 0.53

### 4) H-D-Lys(Boc)-D-Arg(Mtr)NH₂

To a solution of the compound obtained in the preceding step (2.99 g, 4 mmoles) in CH₃OH (50 ml) the addition is performed of a solution of ammonium formate (0.5 g, 8 mmoles) in CH₃OH/H₂O 9/1 (10 ml), and of palladium supported on activated charcoal 10% P (Pd/C) (1.5 g) in small portions. The reaction mixture is then kept at room temperature with stirring under nitrogen blanket for 30 minutes, and then it is filtered on celite. The filtrate is taken to dryness and the residue so obtained is taken with H₂O (70 ml) and then the pH is adjusted to 9 by addition of a 5 % Na₂CO₃ water solution. The water phase is extracted with AcOEt (3 x 70 ml) and the organic phases are collected and dried over MgSO₄ and taken to dryness. 1.67 g of the desired product is obtained with 69 % yield.

The chromatographic analysis does not show traces of impurities.

The HPLC analysis in the conditions of step 1) puts into evidence a single peak with T_{R} = 19.4′.
tlc (CMA) Rf = 0.52.

### 5) Fmoc-D-Asp(OBu^{t})-D-Lys(Boc)-D-Arg(Mtr)-NH₂

A solution of HOBt (467 mg, 3.26 mmoles) in DMF (3 ml) and a solution of DCC (617 mg, 2.99 mmoles) in CH₂Cl₂ (5 ml) are added to a solution of Fmoc-D-Asp(OBu^{t}) (Bachem) (1.23 g, 2.99 mmoles) in CH₂Cl₂/DMF 37/1 (38 ml) cooled to 0°C and kept stirred under nitrogen blanket.

After 30 minutes the temperature is allowed to rise up to the room temperature value and the stirring is continued for 15 minutes. Then a solution of the compound obtained in step 4) (1.67 g, 2.72 mmoles) in CH₂Cl₂ (10 ml) is added.

The reaction mixture is kept stirred at room temperature for 1.5 hours. Then the DCU formed is removed by filtration, the solvent is evaporated off under reduced pressure and the residue is taken with THF (10 ml). The solution is cooled to -15°C and kept at that temperature overnight, then it is filtered again and the THF is removed from the filtrate by evaporation. The oily residue so obtained is taken with 100 ml of AcOEt and the organic solution is washed in succession with a 5 % NaHCO₃ water solution (3 x 50 ml), a NaCl saturated water solution (3 x 50 ml), a 0.1 N HCl water solution (3 x 50 ml), and finally again with the NaCl saturated solution (3 x 50 ml). The organic phase washed is then dried over NgSO₄ and evaporated to dryness. The oily residue is then triturated with hexane, so giving a white, finely divided powder (2.26 g, yield 82 %).
Melting point 142.5°C

The ¹H-NMR and the mass spectrometric analyses confirm the structure assigned to the product.

The HPLC analysis in the conditions of the step 1) puts into evidence a single peak with T_{R} = 30.64
tlc (CMA) Rf = 0.6

### 6) H-D-Asp(OBu^{t})-D-Lys(Boc)-D-Arg-(Mtr)-NH₂

A solution of the compound of the preceding step (2.26 g, 2.24 mmoles) in a 80/20 DMF/diethylamine mixture (25 ml) is stirred at room temperature for one hour under nitrogen blanket. The solvent is removed by evaporation under reduced pressure and the residue is taken with AcOEt (50 ml). The organic solution is extracted with a 0.1 N HCl water solution (3 x 40 ml), the water extracts combined are then adjusted to pH 8 with 1 M NaOH and extracted again with AcOEt (3 x 70 ml). The organic phase is than dried over MgSO₄ and then taken to dryness, leaving behind an oily residue which is triturated with n-hexane. A white, finely divided powder is obtained (1.3 g, 74% yield).

The ¹H-NMR and mass spectrometric analyses confirm the structure assigned to the product.

The HPLC analysis in the conditions of step 1) puts into evidence a single peak with T_{R} = 21.74.

### 7) Z-D-Val-D-Asp(OBu^{t}-D-Lys(Boc)-D-Arg(Mtr)NH₂

A solution of HOBt (283 mg, 1.98 mmoles) in DMF (3 ml) and one of DCC (375 mg, 1.815 mmoles) in CH₂Cl₂ (5 ml) are added to a solution of Z-DVal (Bachem) (456 mg, 1.815 mmoles) in CH₂Cl₂ (12 ml) previously cooled to 0°C and kept under nitrogen blanket. After 30 minutes the temperature is allowed to rise up to the value of room temperature and the solution is kept stirred for additional 30 minutes. Then a solution of the compound obtained in the preceding step (1.3 g, 1.65 mmoles) in a 2/1 CH₂Cl₂/DMF mixture (15 ml) is added.

The precipitate so formed is filtered and recrystallized from isopropanol so that a microcrystalline white solid is obtained in an amount of 733 mg (yield 44 %).

The ¹H-NMR and mass spectrometric analyses confirm the structure assigned to the product.

The HPLC analysis in the conditions of step 1) puts into evidence a single peak with T_{R} = 28.52′.

### 8) H-D-Val-D-Asp(OBu^{t})-D-Lys(Boc)-D-Arg(Mtr)-NH₂

To a solution of the compound obtained in the preceding step (500 mg, 0.49 mmoles) in CH₃OH (10 ml) the addition is performed of ammonium formate (94 mg, 1.49 mmoles) dissolved in CH₃OH (3.5 ml) and H₂O (0.2 ml), and of palladium supported on activated charcoal 10% P (Pd/C) (500 mg) in small portions. The reaction is then carried out at room temperature and under nitrogen blanket. After two hours, the reaction mixture is filtered over celite and the solvent is removed by evaporation under reduced pressure.

The residue is taken with H₂O (40 ml). The water solution is then adjusted to pH 4 with diluted hydrochloric acid, then extracted with AcOEt (3 x 20 ml), and adjusted to pH 8 by the addition of a sodium bicarbonate saturated solution and extracted with AcOEt (3 x 20 ml). The organic extracts are then collected and taken to dryness under reduced pressure;
400 mg of a colourless foamy solid is obtained (yield 92.3 %).

The ¹H-NMR analysis confirms the structure assigned to the product.

The chromatographic analysis in the conditions pointed out in step 1) puts into evidence a single peak with T_{R} = 22.20′

### 9) HO-(RS)-mTyr(OBu^{t})-D-Val-D-Asp(OBu^{t})-D-Lys (Boc)-DArg(Mtr)-NH₂

To a solution of the product obtained in the preceding step (400 mg, 0.45 mmoles) in tetrahydrofuran (15 ml), the following compounds are added in succession:
2,2-dimethyl-5-(4-tert-butoxy)benzyl-1,3-dioxan-4,6-dione ((M)Tyr(Bu^{t})) (165 mg, 0.54 mmoles), N, O-bis-(trimethylsilyl)acetamide (BSA) (0.224 ml, 0.9 mmoles) and trimethylsilyl chloride (TMSC) (0.057 ml, 0.45 mmoles).

After four hours of stirring a further amount of BSA is added (0.044 ml, 0.018 mmoles) and the reaction is continued for additional 20 hours. Then water is added (70 ml) and the solution is extracted with AcOEt (3 x 50 ml). The organic extracts are collected, dried over MgSO₄ and then evaporated under reduced pressure. 270 mg of a colourless oily product is obtained (yield 53.7 %).

The ¹H-NMR and mass spectrometric analyses confirm the structure assigned to the product.

The HPLC analysis in the conditions of step 1) puts into evidence the two peaks of the diastereoisomers at T_{R} = 28.03′ and T_{R} = 28.89′.

### 10) HO-(RS)mTyr(Bu^{t})-D-Val-D-Asp(OBu^{t})-D-Lys(Boc)-DArg(Mtr)-H.CF₃COOH

The compound obtained in the preceding step (270 mg, 0.24 mmoles) is dissolved in a mixture containing CH3CN (3 ml), DMF (1 ml) and H₂O (2.5 ml), kept at room temperature under nitrogen blanket. Then a solution of TIB (122 mg, 0.28 mmoles) in CH₃CN (1 ml) is added. After three hours, a further portion of TIB equal to 20 % (25 mg, 0.6 mmoles) is added and the solution is kept stirred for additional two hours. The reaction mixture is then taken to dryness and the residue is taken with CH₃OH (20 ml) and again taken to dryness several times. The residue so obtained contains the desired product with a purity higher than 85% as determined by HPLC in the conditions of step 1), by which a double peak is put into evidence.

The product is then passed to the next operation with no further purification.

### 11) HO-(RS)mTyr-D-Val-D-Asp-D-Lys-gArg-H.CH₃COOH

### c.r.i. TP5

About 260 mg of the product obtained in the preceding step (about 0.23 mmoles) is treated for 20 minutes at room temperature and under nitrogen blanket with a freshly prepared mixture containing trifluoracetic acid, trifluoromethan-sulfonic acid and 1,2-ethandithiole (TFA/TFMSA/EDT, 89/1/10 (20 ml). After 20 minutes, the reaction mixture is cooled to 0°C, and an amount of TEA (0.3 ml, 1.44 moles) is added dropwise, and then the solution is taken to dryness under nitrogen flow. The residue is taken with H₂O (70 ml), the water solution is exracted with Et₂O (50 ml), the ether phase is then back-washed with H₂O (30 ml). The two water phases are then collected and washed with Et₂O (3 x 50 ml). The water phase is then evaporated off under reduced pressure and the oily residue is purified by ion exchange chromatography over a column (15 x 0.9 cm) packed with CM-Sephadex C-25 (2 g) and developed with a linear gradient of ammonium acetate at pH 5.5 from 0.15 to 0.6 M during 8 hours at a flowrate of 0.8 ml/min.

The fractions containing the desired product are collected, concentrated down to small volume and lyophilized several times.

The product is further chromatographed over Lychroprep RP-18 eluting with 0.05 M CH₃COONH₄, at pH 5.5/CH3CN (92.8 volume/volume), flowrate 8 ml/min.

The fractions containing the desired product are then collected, concentrated to small volume and lyophilized several times.
10 mg of the product is obtained (yield 15%).

The ¹H-NMR and the mass spectrometric analyses confirm the structure of the product, while the HPLC analysis in the standard conditions of step 1), confirms the purity of the same, putting into evidence two only peaks, given by the diastereoisomer pair with T_{R} = 10.69′ and 12.37′.

## Claims

1. A thymopentin analogue having the following general formula 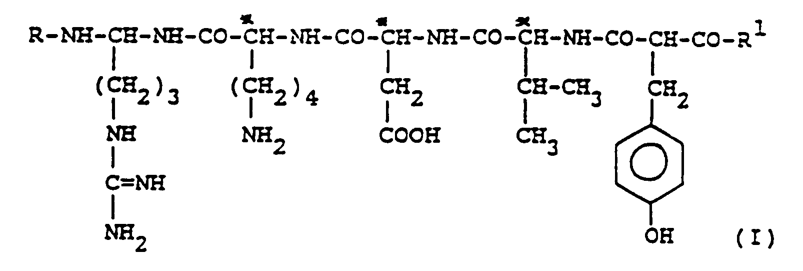 wherein R is hydrogen or an acyl radical which is metabolically labile or whose bond with the amino group is rapidly cleaved in the first steps of the metabolic path of the product,
R¹ is a group -OR² wherein
R² is a hydrogen atom or an alkyl radical having a linear or a branched chain containing 1-6 carbon atoms, a linear or branched chain alkenyl or alkynyl radical containing 3-6 carbon atoms, or an aryl-alkyl or alkyl-aryl radical containing 7-12 carbon atoms,
the configuration of the carbon atoms marked with an asterisk being the D- configuration,
and the corresponding basic or acid addition salts which are pharmaceutically acceptable.

2. A compound according to claim 1, wherein R and R¹ are as defined in claim 1 and R² is hydrogen.

3. A compound according to claim 2, wherein R is hydrogen.

4. A pharmaceutical composition comprising a compound according to claim 1 as the active principle.

5. A compound according to claim 1 for use as a drug.

6. A process for the preparation of a thymopentin analogue having the following general formula: 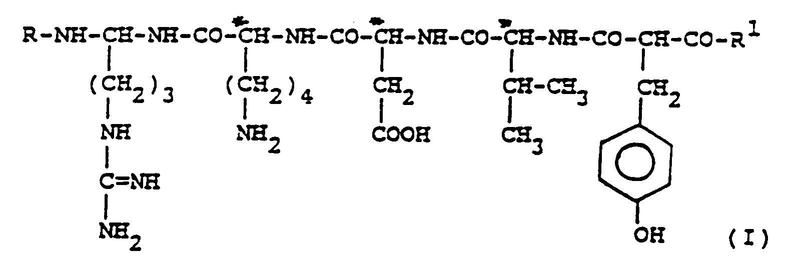 wherein R is hydrogen or an acyl radical which is metabolically labile or whose bond with the amino group is rapidly cleaved in the first steps of the metabolic path of the product,
R¹ is a group -OR² wherein
R² is a hydrogen atom or a linear or branched chain alkyl radical containing from 1 to 6 carbon atoms, a linear or branched chain alkenyl or alkynyl radical containing from 3 to 6 carbon atoms, or an aryl-alkyl or alkyl-aryl radical containing from 7 to 12 carbon atoms, the configuration of the asymmetric carbon atoms marked with an asterisk being the D- configuration, and the corresponding basic or acid addition salts which are pharmaceutically acceptable,
said process consisting in removing the protective groups from the corresponding compound of the formula (Ia) 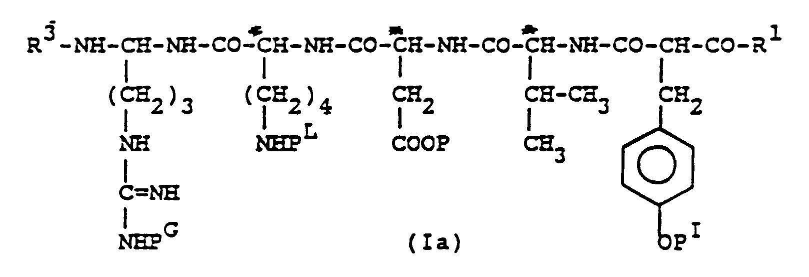 wherein R³ is R or a protective group of the amino function in the alpha-position,
P^{G} is a suitable protective group of the guanidino function,
P^{L} is a protective group of the amino function in the side chain,
P is a protective group of the carboxyl function,
P^{I} is a protective group of the hydroxyl function of tyrosine and
R¹ is as defined above.

7. An intermediate compound of the formula (Ia), (IV) or (VI) 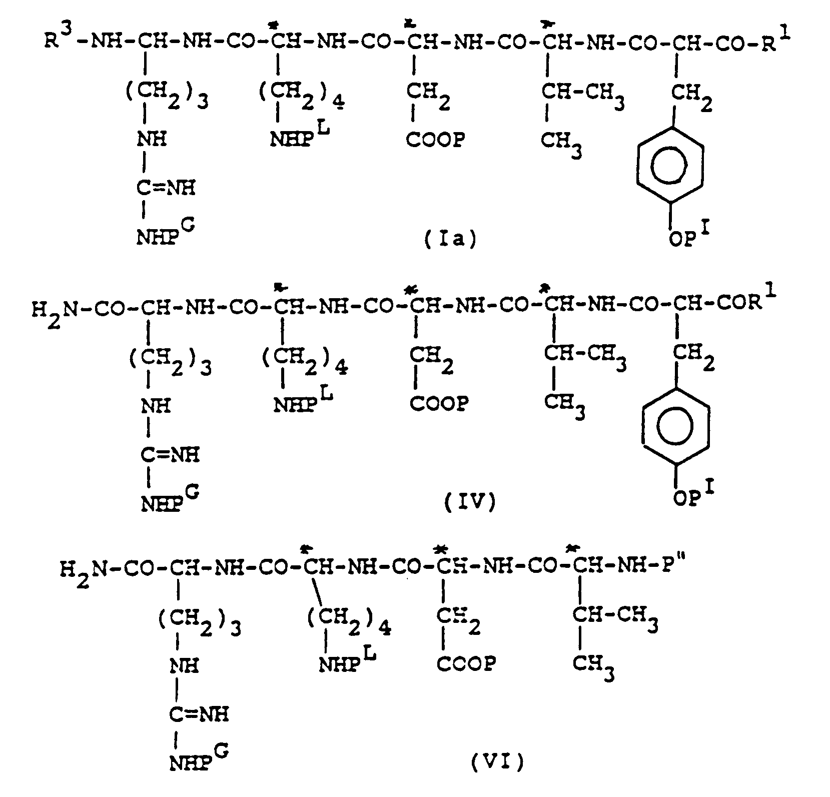 wherein R³ is hydrogen, an acyl group or a protective group of the amino function in the alpha-position,
P^{G} is a protective group of the guanidino function,
P^{L} is a protective group of the amino function in the side chain,
P is a protective group of the carboxyl function,
P^{I} is a protective group of the hydroxyl function of tyrosine,
P˝ is hydrogen or a protective group of the alpha-amino function, and
R¹ is a group -OR² wherein
R² is a hydrogen atom or a linear or branched chain alkyl radical containing from 1 to 6 carbon atoms, a linear or branched chain alkenyl or alkynyl radical containing form 3 to 6 carbon atoms, or an aryl-alkyl or an alkyl-aryl radical containing atoms marked with an asterisk being of the D- configuration.

## Patentansprüche

1. Thymopentin-Analoge der Formel 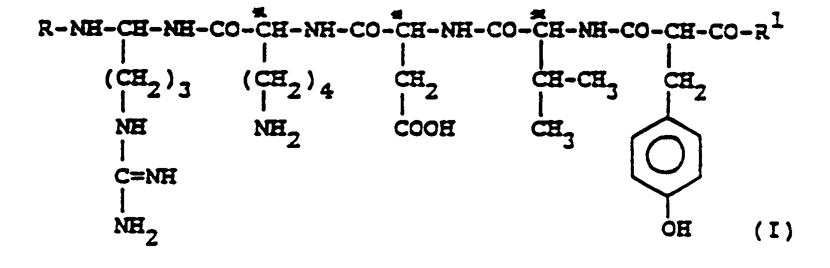 in welcher
R für H oder für ein Acyl-radikal, das metabolisch labil ist, oder dessen Bindung mit der Amino-gruppe schnell in die ersten Stufen des metabolischen Verlaufes der Verbindung gespaltet wird, steht
R¹ für die -OR² Gruppe steht, wobei
R² für H oder für ein geradkettiges oder verzweigtes Alkyl mit 1 bis 6 Kohlenstoffatomen, für ein geradkettiges oder verzweigtes Alkenyl oder Alkynyl mit 3 bis 6 Kohlenstoffatomen, oder für ein Aryl-alkyl oder Alkyl-aryl mit 7 bis 12 Kohlenstoffatomen, steht,
die Konfiguration des mit einem Sternchen markierten Kolhenstoffatoms eine D-Konfiguration ist,
und die pharmazeutisch akzeptabel entsprechenden, basischen oder säurige Additionssalzen.

2. Verbindung gemäß Anspruch 1, wobei R und R¹ die oben angegebene Bedeutung haben und R² Wasserstoff ist.

3. Verbindung gemäß Anspruch 2 wobei R Wasserstoff ist.

4. Pharmazeutisches Mittel wobei die aktive Verbindung eine Verbindung gemäß Anspruch 1 ist.

5. Verbindung gemäß Anspruch 1 als Arzneimittel.

6. Verfahren zur Herstellung von Thymopentin-analoge der Formel 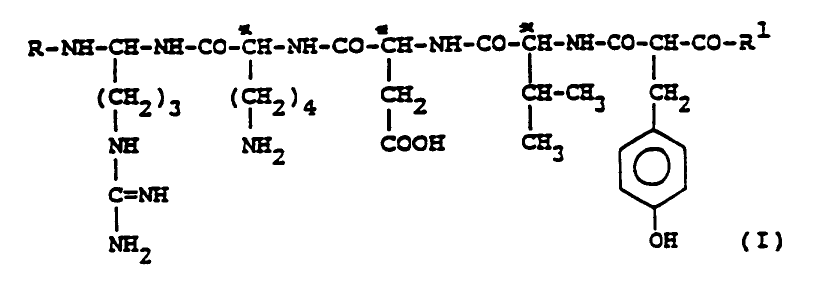 in welcher
R für H oder für ein Acyl-radikal, das metabolisch labil ist, oder dessen Bindung mit der Amino-gruppe schnell in die ersten Stufen des metabolischen Verlaufes der Verbindung gespaltet wird, steht,
R¹ für die -OR² Gruppe steht, wobei
R² für H oder für ein geradkettiges oder verzweigtes Alkyl mit 1 bis 6 Kohlenstoffatomen, für ein geradkettiges oder verzweigtes Alkenyl oder Alkynyl mit 3 bis 6 Kohlenstoffatomen, oder für ein Aryl-alkyl oder Alkyl-aryl mit 7 bis 12 Kohlenstoffatomen, steht,
die Konfiguration des mit einem Sternchen markierten Kolhenstoffatoms eine D-Konfiguration ist,
und die pharmazeutisch akzeptabel entsprechenden, basischen oder säurigen Additionssalzen,
worin die Schutzgruppen der entsprechenden Verbindung der Formel (Ia) 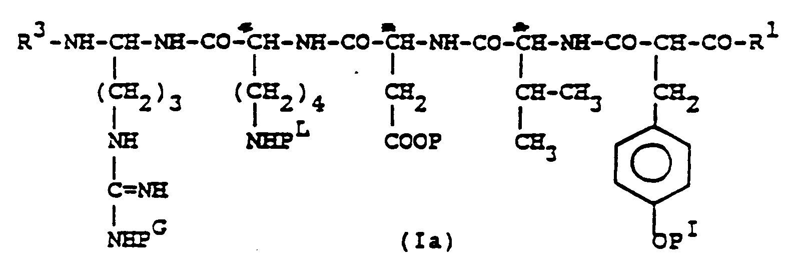 worin R³ R oder eine Schutzgruppe der α-Amino-gruppe ist, abgenommen werden,
P^{G} eine Schutzgruppe der Guanidine-gruppe ist,
P^{L} eine Schutzgruppe der Amino-gruppe in die Seitkette ist,
P eine Schutzgruppe der Carboxyl-gruppe ist,
P^{I} eine Schutzgruppe der Hydroxyl-gruppe der Tyrosine ist,
R¹ die oben angegebene Bedeutung hat.

7. Eine Zwischenverbindung der Formel (Ia) (IV) oder (VI) 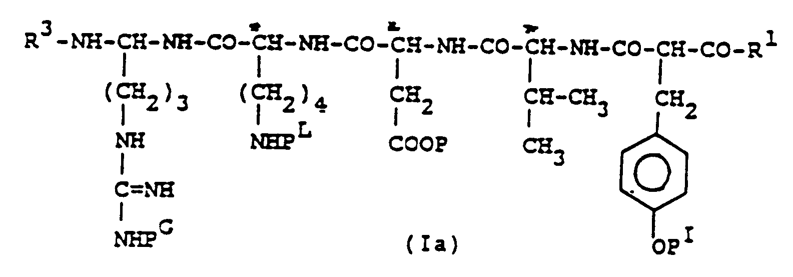 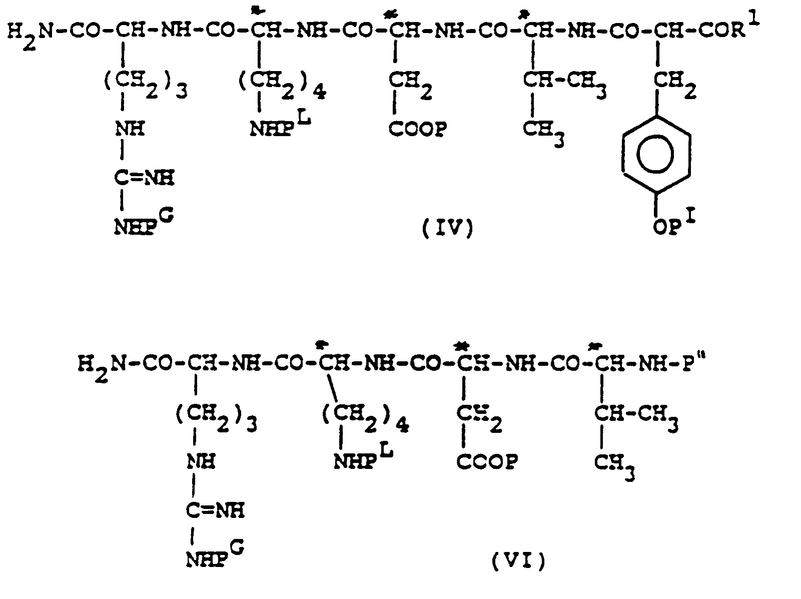 wobei R³ Wasserstoff, eine Acyl-gruppe oder eine Schutzgruppe für die α-Amino-gruppe ist,
P^{G} eine Schutz-gruppe der Guanidine-gruppe ist,
P^{L} eine Schutzgruppe der Amino-gruppe in die Seitkette ist,
P eine Schutzgruppe der Carboxyl-gruppe ist,
P^{I} eine Schutzgruppe der Hydroxyl-gruppe der Tyrosine ist,
P'' Wasserstoff oder eine Schutzgruppe der α-Amino-gruppe ist, und
R¹ eine -OR² Gruppe ist, wobei
R² für H oder für ein geradkettiges oder verzweigtes Alkyl mit 1 bis 6 Kohlenstoffatomen, für ein geradkettiges oder verzweigtes Alkenyl oder Alkynyl mit 3 bis 6 Kohlenstoffatomen, oder für ein Aryl-alkyl oder Alkyl-aryl mit 7 bis 12 Kohlenstoffatomen, steht,
die Konfiguration des mit einem Sternchen markierten Kolhenstoffatoms eine D-Konfiguration ist.

## Revendications

1. Analogue de la thymopentine de formule: 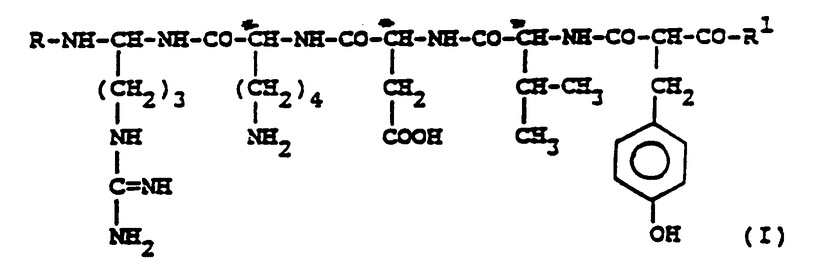 où
R est H ou un radical acyle qui est métaboliquement labile ou formant une liaison avec le groupe amino qui peut être rapidement eliminée dans les premières étapes du procédé métabolique,
R¹ est un groupe -OR² où
R² est H, un radical alkyl linéaire ou ramifié ayant de 1 à 6 atomes de carbone, un radical alkenyl or alkynyl linéaire ou ramifié ayant de 3 à 6 atomes de carbone, ou un radical aryl-alkyl ou alkyl-aryl ayant de 7 à 12 atomes de carbone,
la configuration des atomes de carbone marqués par un astérisque étant une D-configuration
et les correspondant sels de addition acides ou basiques pharmaceutiquement acceptables.

2. Un composé selon la revendication 1 où R et R¹ sont comme définis dans la revendication 1 et R² est H.

3. Un composé selon la revendication 2 où R est H.

4. Une composition pharmaceutique où le composé actif est un composé selon la revendication 1.

5. Un composé selon la revendication 1 comme medicament.

6. Procédé de préparation des analogues de la thymopentine de formule (I) 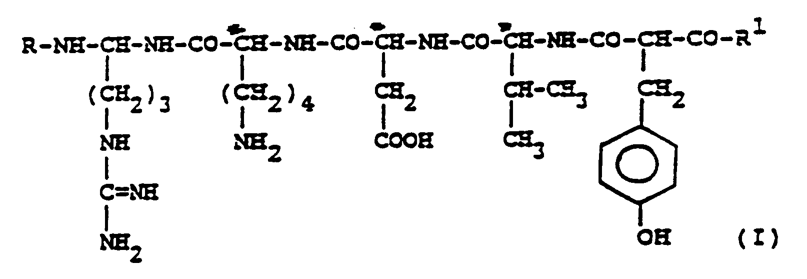 où R est H ou un radical acyle qui est métaboliquement labile ou formant une liaison avec le groupe amino qui peut être rapidement eliminée dans les premières étapes du procédé métabolique,
R¹ est un groupe -OR² où
R² est H, un radical alkyl linéaire ou ramifié ayant de 1 à 6 atomes de carbone, un radical alkenyl or alkynyl linéaire ou ramifié ayant de 3 à 6 atomes de carbone, ou un radical aryl-alkyl ou alkyl-aryl ayant de 7 à 12 atomes de carbone,
la configuration des atomes de carbone marqués par un astérisque étant une D-configuration
et les correspondant sels de addition acides ou basiques pharmaceutiquement acceptables,
où on élimine les groupes protecteurs du correspondant composé de formule (Ia) 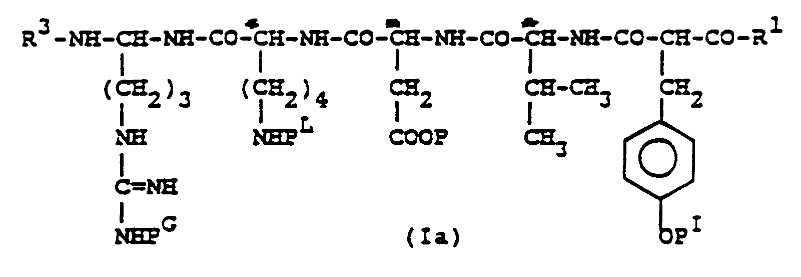 où R³ est R ou un groupe protecteur du groupe amino en position α,
P^{G} est un groupe protecteur du groupe guanidine,
P^{L} est un groupe protecteur du groupe amino de la chaîne latérale,
P est un gropue protecteur du groupe carboxyle,
P^{I} est un groupe protecteur du groupe hydroxyle de la tyrosine et
R¹ est comme précédemment défini.

7. Un composé intermediaire de formule (Ia), (IV) ou (VI) 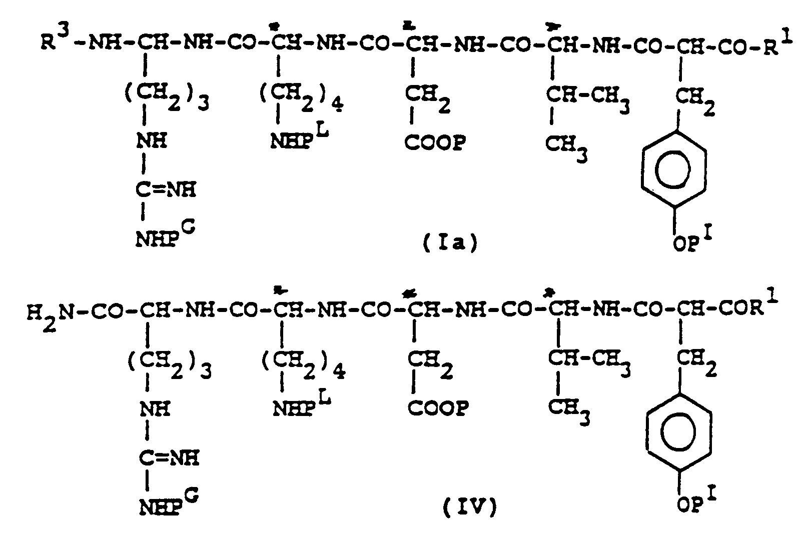 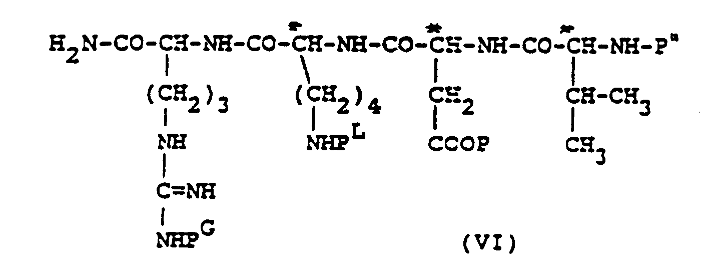 où R³ est H, un groupe acyle ou un groupe protecteur du groupe amino en position α,
P^{G} est un groupe protecteur du groupe guanidine,
P^{L} est un groupe protecteur du groupe amino de la chaîne latéral,
P est un groupe protecteur du groupe carboxyle,
P^{I} est un groupe protecteur du groupe hydroxyle de la tyrosine
P'' est H ou un groupe protecteur du groupe amino en α, et
R¹ est le groupe -OR² où
R² est H, un radical alkyl linéaire ou ramifié ayant de 1 à 6 atomes de carbone, un radical alkenyl or alkynyl linéaire ou ramifié ayant de 3 à 6 atomes de carbone, ou un radical aryl-alkyl ou alkyl-aryl ayant de 7 à 12 atomes de carbone.
la configuration des atomes de carbone marqués par un astérisque étant une D-configuration.
